# EUROPÄISCHE PATENTSCHRIFT

(11) **EP 1 110 510 B1**
(45) Veröffentlichungstag und Bekanntmachung des Hinweises auf die Patenterteilung: **27.03.2002**
(21) Anmeldenummer: 99125925.0
(22) Anmeldetag: 23.12.1999
(51) Int. Cl.: A61B 17/86, A61B 17/88

(54) **Schraube mit dezentralem Antrieb**
Screw driven non-centrally
Vis entraîné hors du centre

(43) Veröffentlichungstag der Anmeldung: 27.06.2001
(73) Patentinhaber: Karl Storz GmbH & Co. KG, 78532 Tuttlingen (DE)
(72) Erfinder: Dr. med. Strobel, Michael, D-94315 Straubing (DE); Dr. Weiler, Andreas, D-10550 Berlin (DE); Timmermans, André, NL-7261 Ruurlo (NL)
(74) Vertreter: Weller, Wolfgang, Dr.

(56) Entgegenhaltungen:
- WO-A-93/21848
- WO-A-96/25104
- DE-A- 3 804 749
- DE-A- 4 131 110
- DE-U- 9 006 908
- FR-A- 2 704 140
- DATABASE WPI Section PQ, Week 9921 Derwent Publications Ltd., London, GB; Class P31, AN 99-247557 XP002137930 & JP 11 070126 A (TAKIRON)

## Beschreibung

Die Erfindung betrifft eine Schraube für den medizinischen Einsatz, mit einem Schraubenkörper, der einen Kopfabschnitt und einen daran anschließenden Schaftabschnitt aufweist, welcher ein Außengewinde aufweist, und mit einer Ausnehmung, an der ein Werkzeug zum Drehen der Schraube ansetzbar ist, wobei die Ausdehnung in Form zumindest einer Längsnut ausgebildet ist, die von der Außenseite her in den Schraubenkörper eingeschnitten ist.

Die Erfindung betrifft ferner ein Werkzeug zum Drehen einer solchen Schraube.

Eine derartige Schraube ist aus der WO-A-96/25 104 bekannt.

In den Schraubenkörper sind sowohl im Kopfabschnitt als auch im daran anschließenden Schaftabschnitt Ausnehmungen in Form einer Längsnut eingeschnitten, in die ein Werkzeug zum Drehen der Schraube einsetzbar ist. Die Ausnehmungen erstrecken sich dabei über eine gewisse Länge des Schaftabschnittes.

Derartige Schrauben sind allgemein aus OP-Journal 14 (1998), Seite 278 - 284 "Biodegradierbare Interferenzschrauben in der Kreuzbandchirurgie", A. Weiler et al., Georg Thieme Verlag, Stuttgart, New York, bekannt.

Interferenzschrauben dienen insbesondere dazu, ein Sehnentransplantat oder ein Sehnenimplantat in einem Knochen zu verankern. Dazu wird in den Knochen ein Kanal gebohrt, in den das Sehnentransplantat eingezogen wird. Die Schraube ist nun dazu vorgesehen, in einen Zwischenraum zwischen Sehnentransplantat oder Innenwand des Bohrkanals eingedreht zu werden, so daß dann das Sehnentransplantat zwischen der Schraube und der Innenwand des Bohrkanals geklemmt ist. Da auf eine solche Sehne, bspw. bei einem Kreuzbandersatz in einem Kniegelenk, erhebliche Kräfte einwirken, muß die Klemmkraft entsprechend groß sein, um eine dauerhafte Verankerung sicherzustellen. Dazu ist die Schraube mit einem Außengewinde versehen, das sich in das Knochenmaterial in der Innenseite des Bohrkanals hineinfrißt. Gleichzeitig tritt das Außengewinde auch in Eingriff mit dem zu verankernden Sehnentransplantat.

Es gibt auch andere Einsatzgebiete für Schrauben im chirurgischen Bereich zum Verbinden von Materialien.

Zum Drehen der Schraube ist im Kopf mittig eine Ausnehmung vorgesehen, in die ein Werkzeug, bspw. ein entsprechender Schraubendreher, eingesetzt werden kann. Da erhebliche Kräfte, insbesondere bei den letzten Umdrehungen vor dem endgültigen Sitz, auf die Schraube einwirken, ist vorgesehen, diese Ausnehmung nicht nur im Bereich des Kopfes bei einem nur am Kopf angreifenden Antrieb zu bewerkstelligen, sondern zentral mittig axial weit in den Schaftbereich hineinreichen zu lassen. Die Kräfte werden daher vom Werkzeug nicht nur auf den Kopf abgeleitet, sondern über große Bereiche des Schraubenkörpers verteilt. Dies insbesondere deswegen, um ein Abscheren des Kopfes vom verbleibenden Schraubenkörper zu verhindern, insbesondere bei den letzten Drehbewegungen vor dem endgültigen Sitz.

Es ist bekannt, solche Schrauben aus metallischen Materialien insbesondere aus Titan herzustellen, es haben aber verbreitet auch Schrauben aus biodegradierbaren Materialien Einsatz gefunden. Es wurde festgestellt, daß biodegradierbare Schrauben aus geeigneten Materialien eine gleich hohe initiale Verankerungsfestigkeit wie Metallschrauben aufweisen.

Allerdings bestehen erhebliche Schwierigkeiten beim Eindrehen der Schrauben.

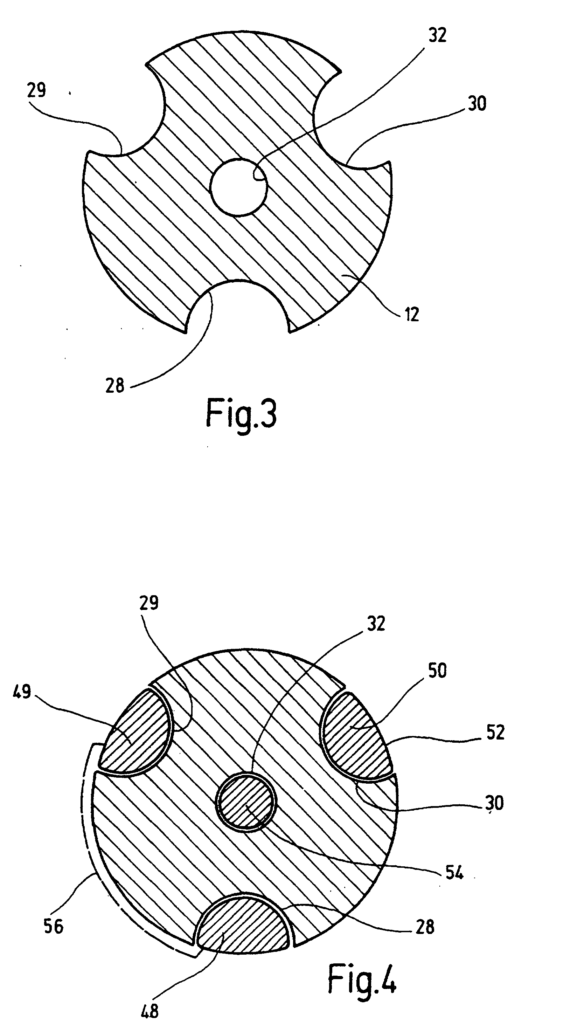

Weist die zentrale Ausnehmung die Form eines Innensechskants auf, in den ein Werkzeug in der Form eines Inbusschlüssels eingesetzt werden kann, besteht insbesondere bei biodegradierbaren Materialien die Gefahr, daß die Sechskantkontur ausgerissen wird und das Werkzeug sich dann ohne Kraftschluß im Schraubenkörper dreht. Daher wurde versucht, andere Querschnittsformen des mittigen zentralen Antriebes zu wählen, die einen großflächigeren Eingriff mit dem Werkzeug ermöglichen, so sind insbesondere aus Abbildung 3 der eingangs erwähnten Veröffentlichung im OP-Journal solche Querschnittskonturen ersichtlich, bspw. in Kreuzform oder in Form einer Turbine. Daraus ist eine Tendenz zu erkennen, die mittige Ausnehmung relativ groß und verästelt herzustellen, so daß nur noch ein relativ dünnwandiger Schraubenkörper verbleibt.

Dies hat nun den Nachteil, daß insbesondere bei kleinen und dünnen Schrauben ein äußerst dünnwandiger Schraubenkörper vorhanden ist, dessen Verwindungssteifigkeit dann nicht mehr ausreichend ist, um den hohen Kräften beim Eindrehen zu widerstehen.

Ferner führt diese Tendenz der immer größeren mittigen zentralen Ausnehmung dazu, daß ein Schraubenkörper mit einem relativ großen mittigen Hohlvolumen entsteht. Dies hat insbesondere bei biodegradierbaren Schrauben den Nachteil, daß, wenn die Wand des dünnwandigen biodegradierbaren Körpers nach relativ kurzer Zeit biologisch abgebaut ist, ein relativ großer Hohlraum entsteht, so daß dann keine ausreichende Klemmkraft mehr vorhanden ist, um das Sehnentransplantat an Ort und Stelle zu halten.

Es ist daher Aufgabe der vorliegenden Erfindung, hier Abhilfe zu schaffen und eine Schraube zu schaffen, die auch bei kleiner und dünner Bauweise hohe Kräfte beim Drehen der Schraube sicher aufnehmen kann, ohne daß Verformungen oder Ausrisse auftreten, und an der das Werkzeug exakt ausgerichtet angesetzt werden kann.

Erfindungsgemäß wird die Aufgabe bei einer Schraube dadurch gelöst, daß sich die zumindest eine Längsnut über die gesamte Länge des Schaftabschnittes erstreckt, und daß am stirnseitigen Ende des Kopfabschnittes zumindest eine Aussparung vorgesehen ist, in die ein entsprechender Vorsprung am Werkzeug eintreten kann.

Die Maßnahme, daß sich die zumindest eine Längsnut über die gesamte Länge des Schaftabschnittes erstreckt, hat den Vorteil, daß die Kräfte des Werkzeugs über nahezu die gesamte axiale Länge des Schraubenkörpers, auf jeden Fall über die gesamte axiale Länge des Schaftabschnittes, verteilt werden können.

Das Vorsehen einer Aussparung am stirnseitigen Ende des Kopfabschnittes hat den Vorteil, daß diese Aussparung im Kopf als Ansatz- oder Zentrierhilfe für das Werkzeug dienen kann. Dies führt zu einem exakt ausgerichteten Sitz des Werkzeuges an der Schraube.

In einer weiteren Ausgestaltung der Erfindung ist vorgesehen, die Einschnittiefe der zumindest einen Längsnut derart auszugestalten, daß ein Arbeitselement des Werkzeuges innerhalb der Längsnut zum Liegen kommt und die umfängliche Außenkontur der Schraube nicht oder nur unwesentlich überragt.

Diese Maßnahme hat nun den erheblichen Vorteil, daß ein an die Schraube angelegtes Werkzeug die umfängliche Außenkontur nicht überragt, so daß im wesentlichen nur das Außengewinde des Schraubenkörpers mit dem Sehnentransplantat und der Innenwand der Bohrung des Knochens in Berührung tritt und nicht auch noch das Werkzeug. Das hat insbesondere den Vorteil, daß nach Abziehen des Werkzeuges umfänglich um den Schraubenkörper herum keine Hohlräume entstehen, die beim Eindrehen durch das Werkzeug belegt waren, in die dann nach Abziehen des Werkzeuges Knochen bzw. Transplantatmaterial zur festen Verankerung nachrücken müßten.

Überragt das Werkzeug die Schraube umfänglich nur in geringem Maße oder nur an einigen Stellen, also wenn nur eine oder zwei Längsnuten vorhanden sind, so kann auch mit einem solchen Werkzeug, das die Längsnuten umfänglich überragt, eine Schraube festsitzend eingedreht werden. Insbesondere deswegen, da das poröse Knochenmaterial und auch das Material des Sehnentransplantates eine gewisse Flexibilität aufweisen, so daß nach Setzen der Schraube zum einen das Werkzeug abgezogen werden kann und zum anderen in die dadurch geschaffenen Freiräume das Material nachrücken kann und für eine zusätzliche feste Verankerung der Schraube sorgt.

In einer weiteren Ausgestaltung der Erfindung ist vorgesehen, daß die Aussparung als durch den Schraubenkörper durchgehender Kanal ausgebildet ist.

Dies hat insbesondere bei relativ großen Schrauben den Vorteil, daß in den inneren Hohlraum während des biologischen Abbaus des diesen Hohlraum umgebenden Körpers schon über den Kanal Material hineinwachsen kann. Insbesondere bei Ausgestaltungen, bei denen von der Außenseite in den Körper zusätzliche Öffnungen oder Löcher vorgesehen sind, die in dem mittigen Kanal münden, ist ein gleichzeitiges Hineinwachsen von Material in den Schraubenkörper parallel zu dessen biologischem Abbau möglich.

In einer weiteren Ausgestaltung ist vorgesehen, daß mehrere, gleichzeitig um den Außenumfang des Schraubenkörpers verteilt angeordnete Längsnuten vorgesehen sind.

Diese Maßnahme hat den Vorteil, daß die Kräfte nicht nur über eine einzige Längsnut axial sondern durch die mehreren umfänglich angeordneten Längsnuten auch noch gleichmäßig umfänglich verteilt auf den Schraubenkörper einwirken, so daß bei sehr dünnen Schraubenkörpern dann extrem hohe Drehkräfte gleichmäßig auf den Körper verteilt werden können, wodurch die Gefahr von Deformationen oder Abscheren durch punktuelle Kraftspitzen ausgeschlossen ist.

In einer weiteren Ausgestaltung ist vorgesehen, daß eine bis fünf, insbesondere drei Längsnuten eingeschnitten sind.

Diese Anzahl an Längsnuten erlaubt im Rahmen der üblichen Größen solcher Schrauben eine optimale Kraftverteilung bei dünnen Schrauben und dennoch ausreichend stabilem Werkzeug.

In einer weiteren Ausgestaltung der Erfindung ist vorgesehen, daß die Längsnut axial am stirnseitigen Ende des Kopfabschnitts offen ist.

Diese Maßnahme hat den Vorteil, daß das Werkzeug axial auf die Schraube aufgeschoben und, insbesondere nach Eindrehen der Schraube axial über diese Öffnung von der Schraube wieder abgezogen werden kann. Insbesondere in Zusammenhang mit der zuvor erwähnten Ausgestaltung, wonach das Werkzeug die Kontur der Schraube umfänglich nicht überragt, führt dies vorteilhaft zu einem sanften Abziehen des Werkzeuges nach Eindrehen der Schraube, ohne dabei das die Schraube umgebende Material, also Knochenmaterial und Sehnentransplantat zu beeinträchtigen.

In einer weiteren Ausgestaltung ist vorgesehen, daß die Längsnut, insbesondere im Kopfabschnitt, umfänglich mit zumindest einem Steg überbrückt ist.

Diese Maßnahme hat den Vorteil, daß, nachdem das Werkzeug auf die Schraube aufgeschoben ist, der Steg verhindert, daß das Werkzeug seitlich bzw. in radialer Richtung von der Schraube abgleitet. Dies ist insbesondere von Vorteil, wenn nur eine einzige äußere Längsnut vorhanden ist. Da üblicherweise bei solchen Schrauben im Kopfbereich kein Außengewinde eingeschnitten ist, ist im Bereich des Kopfes ein höherer Materialdurchmesser vorhanden, so daß, auch bei eingeschnittener Längsnut, noch möglich ist, diese über einen äußeren umfänglichen Steg abzuschließen.

Ein solcher Steg kann auch durch die von der Außenseite des Schraubenkörpers vorstehenden Gewindegänge gebildet werden. Bei manchen Schraubentypen sind relativ große Gewinde vorhanden, so daß ein ausreichend fester Materialsteg verbleibt.

Erfindungsgemäß trägt zur Lösung der Aufgabe auch ein Werkzeug zum Drehen einer Schraube bei, das zumindest ein Arbeitselement aufweist, das in eine entsprechende Längsnut an der Schraube einsetzbar ist, und bei dem distalseitig zumindest ein Vorsprung vorsteht, der in die Aussparung am stirnseitigen Kopfabschnitt der Schraube eintreten kann.

Dadurch ist ein exakt auf die Konstruktion der jeweiligen Schraube bzw. die Ausgestaltung deren Längsnut angepaßtes Werkzeug vorhanden, also bspw. ein an die Anzahl, die Geometrie und die Länge der Längsnut angepaßtes Werkzeug, so daß ein optimales Zusammenwirken zwischen Werkzeug und Schraube bei der Handhabung gewährleistet ist. Das Vorsehen des vorstehenden Vorsprunges, der in die Aussparung am stirnseitigen Kopfabschnitt der Schraube eintreten kann, hat den erheblichen Vorteil, daß dieser Vorsprung als Zentrierhilfe beim Ansetzen der Schraube an dem Werkzeug dienen kann. Hat die Schraube nur eine einzige Längsnut, kann ein in die Aussparung am Kopf der Schraube eintretender Vorsprung des Werkzeuges noch als zusätzliche Sicherung dagegen dienen, daß beim Drehen des Werkzeuges dieses sich aus der Schraube herausdreht.

In einer weiteren Ausgestaltung des Werkzeuges ist das zumindest eine Arbeitselement derart ausgestaltet, daß dies in Längsrichtung von der Kopfseite her in die entsprechende Längsnut einschiebbar ist.

Diese Maßnahme hat den Vorteil, wie zuvor erwähnt, daß zur Handhabung das Werkzeug auf die Schraube aufgesetzt bzw. umgekehrt, bei in einer Hand gehaltenem Werkzeug die Schraube auf dieses aufgesteckt werden und dann an der entsprechenden Stelle, an der die Schraube eingedreht werden soll, angesetzt werden kann. Dabei können dann die Toleranzen zwischen Breite der Längsnut und Breite des entsprechenden in die Längsnut eintretenden Arbeitselementes so gewählt sein, daß die Schraube fest am Werkzeug sitzt, was die Handhabung für den Operateur erleichtert, er also nicht Sorge tragen muß, daß beim Ansetzen die Schraube vom Werkzeug abfällt.

Bei der Ausgestaltung, wonach die Längsnut, insbesondere im Kopfabschnitt, umfänglich mit zumindest einem Steg überbrückt ist, besteht, wie zuvor erwähnt, insbesondere in Zusammenhang mit einer Schraube mit nur einer einzigen Längsnut der Vorteil, daß ein seitliches Abfallen oder Abkippen der Schraube durch den Steg gehindert ist.

In einer weiteren Ausgestaltung beim Vorhandensein von mehreren Arbeitselementen ist vorgesehen, die Arbeitselemente über umfängliche Stege miteinander zu verbinden.

Diese Maßnahme hat den Vorteil, daß ein radiales Aufspreizen bei langen und dünnen Arbeitselementen verhindert wird. Es können Sollbruchstellen vorgesehen sein, so daß beim Abziehen des Werkzeuges sich die Stege von den Arbeitselementen lösen und an der Schraube verbleiben. Dadurch kann das Werkzeug einfach von der eingedrehten Schraube abgezogen werden.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Seitenansicht einer erfindungsgemäßen Schraube mit drei gleichmäßig umfänglich verteilten Längsnuten,
- Fig. 2: eine der Darstellung von Fig. 1 entsprechende Ansicht mit einem Werkzeug, das gerade auf die Schraube aufgeschoben wird,
- Fig. 3: einen Schnitt längs der Linie III-III in Fig. 1,
- Fig. 4: einen Schnitt längs der Linie IV-IV in Fig. 2,
- Fig. 5: einen dem Schnitt von Fig. 3 vergleichbare Darstellung eines weiteren Ausführungsbeispiels einer Schraube,
- Fig. 6: einen der Fig. 5 bzw. der Fig. 3 entsprechenden Schnitt eines weiteren Ausführungsbeispiels einer Schraube,
- Fig. 7: eine Seitenansicht eines Werkzeuges, das mit der in Fig. 6 dargestellten Schraube zusammenwirkt,
- Fig. 8: eine teilweise geschnittene Ansicht einer Schraube entsprechend der Ausgestaltung von Fig. 6, auf die ein Werkzeug entsprechend der Darstellung von Fig. 7 zu Beginn eines Eindrehvorganges beim Fixieren eines Sehnentransplantates aufgesetzt ist, und
- Fig. 9: die in Fig. 8 dargestellte Schraube nach vollständigem Eindrehen mit verankertem Sehnentransplantat.

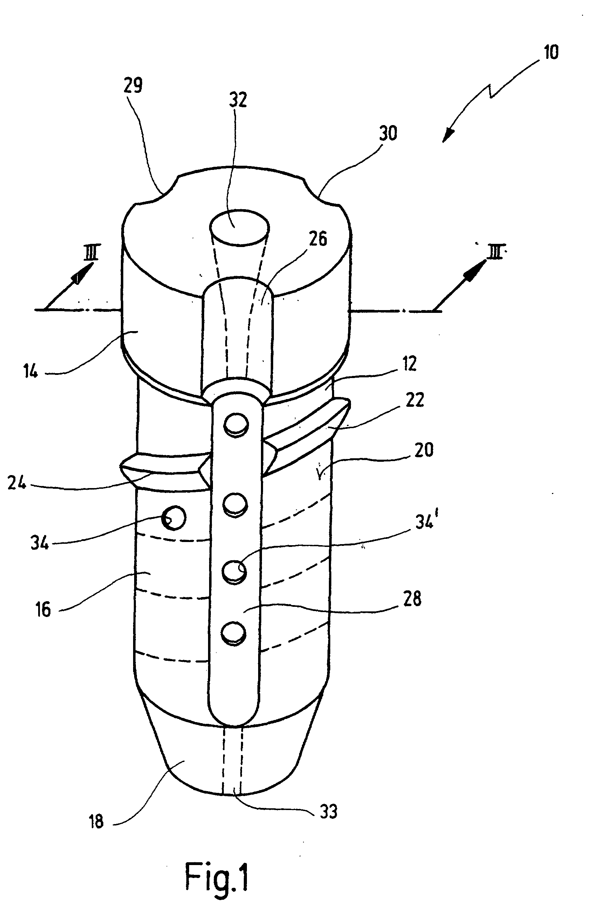

Eine in Fig. 1 dargestellte Schraube ist in ihrer Gesamtheit mit der Bezugsziffer 10 bezeichnet und ist als Interferenzschraube ausgebildet.

Die Interferenzschraube 10 weist einen Interferenzschraubenkörper 12 auf, der einen Kopfabschnitt 14 und einen daran anschließenden Schaftabschnitt 16 aufweist, der in einem sich verjüngenden Eintreibende 18 endet. Die Interferenzschraube 10 kann aus Metall hergestellt sein, bspw. aus Titan, sie kann aber höchst vorzugsweise aus biodegradierbaren Materialien hergestellt sein. Beispiele für biodegradierbare Materialien sind: Polycaprolactone, Poly(L-Laktide), Polyglykolide, Poly(D,L-Laktide), Poly(D,L-Laktid-co-Glykolide), Poly(D,L-Laktid-co-Caprolactone), Polydioxanone, Copolyoxalate, Polycarbonate, z.B. Polyglykolid-co-Trimethylencarbonat, Poly(Glutamin-co-Leucine).

Die Interferenzschraube 10 ist im Bereich ihres Schaftabschnittes 16 an ihrer Außenseite 20 mit einem Außengewinde 22 versehen.

In der Darstellung von Fig. 1 ist lediglich ein Gang 24 des Auβengewindes 22 dargestellt, die anderen Gänge sind nur durch gestrichelte Linien angedeutet. An der Außenseite 20 ist eine erste Ausnehmung 26 in Form einer axial verlaufenden Längsnut 28 vorhanden. Die Einschnittiefe der Längsnut 28 ist dabei derart, daß diese von der Außenseite her sowohl in den Kopfabschnitt 14 als auch in den Schaftabschnitt 16 eingeschnitten ist. Da im Bereich des Schaftabschnittes 16 das Außengewinde 22 vorgesehen ist, schneidet die Längsnut 28 somit auch das Außengewinde 22, wie das bei dem Gang 24 ersichtlich ist.

Die Einschnittiefe und die Form ist insbesondere aus der Schnittdarstellung von Fig. 3 ersichtlich, d.h. die Längsnut 28 hat die Querschnittsform eines Kreisabschnittes.

Die Längsnut 28 erstreckt sich also vom stirnseitigen Ende des Kopfabschnittes 14 über den gesamten Schaftabschnitt 16 bis hin zum sich verjüngenden Eintreibende 18.

Die Längsnut 28 ist am stirnseitigen Ende des Kopfabschnitts 14 offen.

Umfänglich symmetrisch zur Längsnut 28 sind noch zwei weitere Längsnuten 29 und 30 vorgesehen, die identisch ausgebildet sind wie die Längsnut 28.

Der umfängliche Winkelabstand der drei Längsnuten 28, 29 und 26 untereinander ist somit 120°.

Mittig im Kopfabschnitt 14 ist eine Aussparung 32 vorgesehen, die in dem dargestellten Ausführungsbeispiel über einen konisch sich verjüngenden Abschnitt in einen dünnen mittig durchgehenden Kanal 33 übergeht, der bis in das Eintreibende 18 reicht und dort zur Außenseite mündet.

Im Schraubenkörper 12 sind ferner zahlreiche axial ausgerichtete Bohrungen oder Löcher 34, 34' vorgesehen, die in den mittigen zentralen Kanal 33 münden.

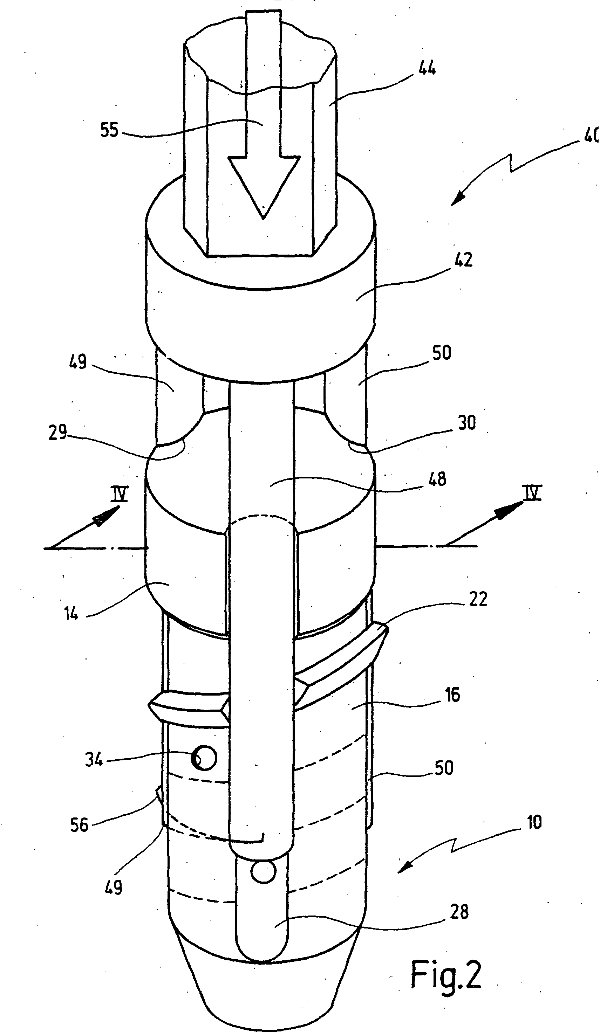

Die zentrale mittige Aussparung 32 dient dazu, um für ein Werkzeug 40, wie es in Fig. 2 dargestellt ist, eine Zentrierhilfe darzustellen. Dazu ist am Werkzeug 40 ein Vorsprung 54 vorgesehen (siehe insbesondere Schnittdarstellung von Fig. 4), der zapfenartig ausgebildet ist und in die Aussparung 32 einfahren kann.

Das Vorsehen der Öffnungen 34, 34', die mit dem mittigen Kanal 33 in Verbindung stehen, hat den Zweck, daß, wenn die Interferenzschraube 10 aus biodegradierbarem Material besteht, über diese Öffnungen in den Innenraum biologisches Material hineinwachsen kann. Dadurch entsteht dann ein mit biologisch gewachsenem Material eng verästelter Körper, dessen abbaubares Material sich nach und nach biologisch abbaut. Da das Einwachsen des biologischen Materials meist schneller verläuft als der biologische Abbau, ist also schon ein inniger räumlich durchsetzter und durchnetzter Verbund zwischen dem die Interferenzschraube umgebenden biologischen Material und dessen Innenraum gegeben, und zwar zu einem Zeitpunkt, bevor das biodegradierbare Material des Schraubenkörpers 12 abgebaut ist.

Dies eröffnet insbesondere bei Revisionen die Möglichkeit, im Bereich der Interferenzschraube 10 eine weitere Bohrung anzusetzen, da dann dort schon ausreichendes biologisches Material vorhanden ist, in das ein neues Sehnentransplantat eingesetzt werden kann.

Aus der Darstellung von Fig. 2 und der Schnittdarstellung von Fig. 4 ist ersichtlich, daß ein Werkzeug 40 zum Eindrehen der Interferenzschraube 10 einen Kopf 42 aufweist, von dem proximalseitig ein Griff 44 vorsteht.

Von einer Unterseite 46 des Kopfes 42 stehen drei Arbeitselemente 48, 49, 50 vor.

Die umfängliche Anordnung, Ausgestaltung und Länge der drei Arbeitselemente 48, 49 und 50 ist derart, daß diese in die drei Längsnuten 28, 29 und 30 passend eintreten können.

Die Querschnittskontur 52 eines Arbeitselementes, bspw. des in Fig. 4 dargestellten Arbeitselementes 50, ist derart, daß es gerade den lichten Innenraum der entsprechenden Längsnut 30 ausfüllt. Dies gilt entsprechend für die anderen Arbeitselemente 48 und 49.

Daraus resultiert dann, daß die Arbeitselemente 48, 49 und 50, wenn sie in die Längsnuten 28, 29 und 30 eingetreten sind, nicht über die Umfangskontur des Schraubenkörpers 12 hinausreichen.

Zur Handhabung wird das Werkzeug 40, wie das in Fig. 2 dargestellt ist, von proximal her über den Kopfabschnitt 14 auf die Interferenzschraube 10 aufgeschoben, und zwar in einer solchen Ausrichtung, daß die drei Arbeitselemente 48, 49 und 50 axial in die Längsnuten 28, 29 und 30 einfahren. Der mittige Vorsprung 54 an der Unterseite des Kopfes 42 des Werkzeuges 40 (in Fig. 2 durch das Arbeitselement 48 verdeckt) ist etwas kürzer als die Arbeitselemente 48, 49 und 50 und tritt, etwa in der in Fig. 2 dargestellten Aufschubposition in die Aussparung 32 am proximalseitigen Kopfende der Interferenzschraube 10 ein und sorgt dann für eine ausreichende Zentrierung. Dabei wird das Werkzeug 40 wie in Fig. 2 angedeutet längs eines Pfeiles 45 so weit vorgeschoben, bis die Unterseite 46 des Werkzeuges 40 auf der äußeren Stirnseite des Kopfabschnittes 14 zum Liegen kommt.

Aus der Darstellung von Fig. 2 ist zu sehen, daß dabei die Arbeitselemente 48, 49, 50 zahlreiche Löcher 34' abdecken, so daß beim Eindrehen der Interferenzschraube sichergestellt ist, daß die Löcher zum einen keinen zusätzlichen Widerstand darstellen bzw. darüber nicht unerwünschte Materialien schon beim Eindrehen eintreten.

Durch die lange axiale Länge der drei Arbeitselemente 48, 49 und 50 und dem passenden Sitz in den Längsnuten 28, 29 und 30 ist ein relativ großflächiger kraftschlüssiger Eingriff zwischen dem Werkzeug 40 und der Interferenzschraube 10 vorhanden, so daß auch bei relativ kleinen und dünnen Interferenzschrauben erhebliche Drehkräfte sicher übertragen werden können. Es kann vorgesehen sein, einzelne oder auch alle Arbeitselemente umfänglich untereinander zu verbinden, um ein radiales Spreizen zu verhindern, wie dies in den Fig. 2 und 4 durch einen Steg 56 angedeutet ist, der die Arbeitselemente 48 und 49 umfänglich im distalen Endbereich verbindet. Der Steg 56 ist so ausgestaltet, daß er ein Aufschieben oder Abziehen des Werkzeuges 40 nicht hindert. Ein solcher Steg kann auch durch einen oder mehrere Gänge 24 des Außengewindes 22 gebildet sein, wenn bspw. der in Fig. 1 gezeigte Gang 24 sich quer über die Längsnut 28 fortsetzt.

Nach Eindrehen der Interferenzschraube kann das Werkzeug 40 entgegen der Richtung des Pfeiles 55 von Fig. 2 wieder einfach abgezogen werden, wie das nachfolgend noch anhand eines weiteren Ausführungsbeispieles beschrieben wird.

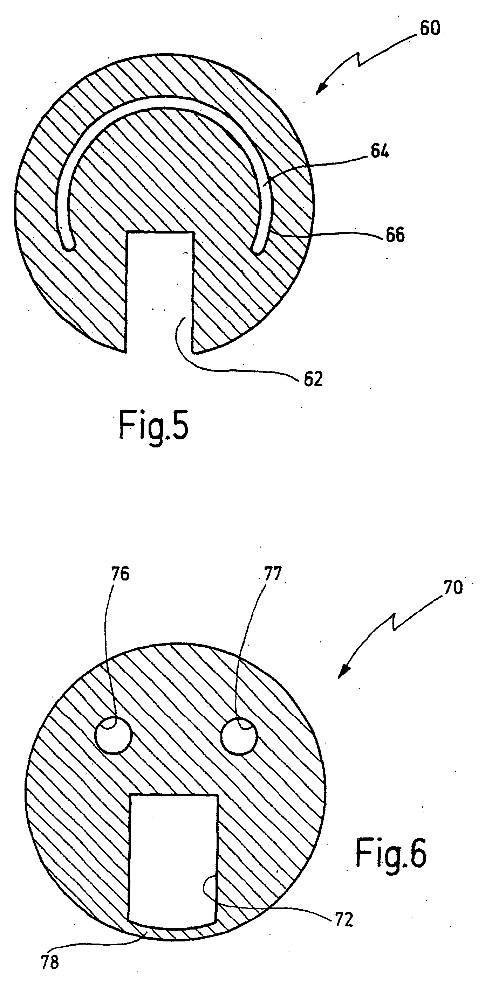

In Fig. 5 ist ein weiteres Ausführungsbeispiel einer Interferenzschraube 60 dargestellt, bei der an deren Außenseite nur eine einzige Längsnut 62 eingeschnitten ist.

Eine Aussparung 64 in Form eines umfangsabschnittsförmigen Schlitzes 66 dient dabei wieder als Ansatz oder Zentrierhilfe für ein entsprechend ausgestaltetes Werkzeug.

Die Einschnittiefe und Breite der Längsnut 62 ist etwas tiefer als dies bei den drei Längsnuten bei der Ausgestaltung von der Interferenzschraube 10 war, der tiefe Einschnitt läßt aber nach wie vor mittig noch einen solchen kompakten festen Körper der Interferenzschraube 16 stehen, daß auch bei erheblichen Drehkräften kein Verwinden oder Abscheren der Interferenzschraube 60 stattfinden kann. Auch hier erstreckt sich die Längsnut 62 durch den Kopfabschnitt und einen weiten Bereich des Schaftabschnittes der Interferenzschraube 60.

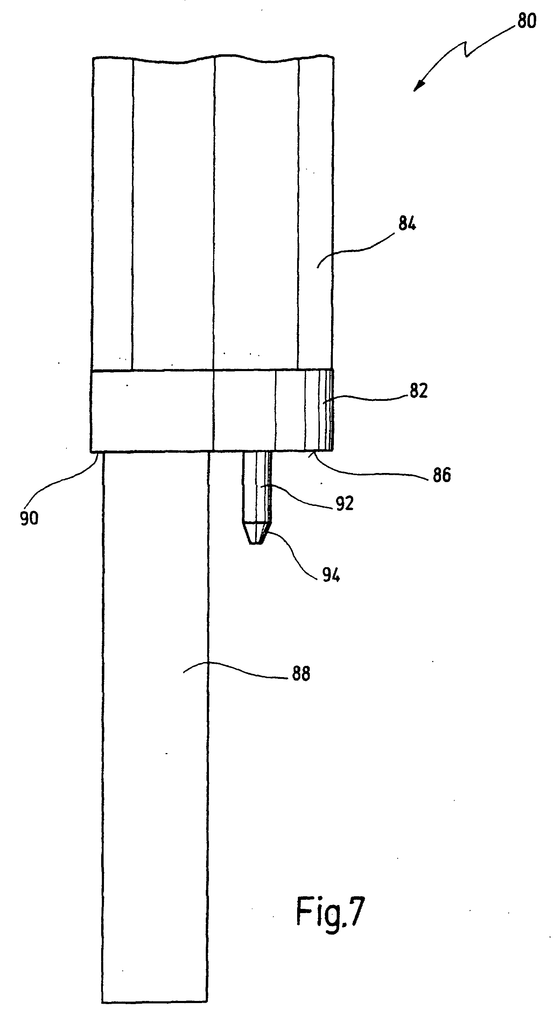

In Fig. 6 ist ein weiteres Ausführungsbeispiel einer Interferenzschraube 70 dargestellt, die ebenfalls, wie zuvor in Zusammenhang mit der Interferenzschraube 60 beschrieben, nur eine einzige Längsnut 72 aufweist. Auch hier ist im Kopfabschnitt der Interferenzschraube eine Aussparung 74 als Zentrierhilfe für das entsprechende Werkzeug 80 vorgesehen, das in Fig. 7 dargestellt ist, wobei dies in Form von zwei Sacklöchern 76 und 77 besteht.

Aus der Schnittdarstellung von Fig. 6 ist ersichtlich, daß die Längsnut 72, hier im Bereich des Kopfabschnittes, umfänglich über einen äußeren Steg 78 verschlossen ist. Das entsprechende Werkzeug 80, das in Fig. 7 dargestellt ist, weist dazu einen Kopf 82 auf, von dem proximalseitig ein Griff 84 zur Handhabung vorsteht. Von der Unterseite 86 des Kopfes 82 steht ein Arbeitselement 88 vor, das einen etwa rechteckförmigen Querschnitt aufweist und dessen Querschnittskontur der Querschnittskontur der Längsnut 72 entspricht. An der äußeren Umfangsseite ist das Arbeitselement 88 etwas zurückgesetzt, so daß eine Stufe 90 entsteht, wobei der Rücksatz der radialen Breite des Steges 78 entspricht. Von der Unterseite 86 springen Vorsprünge 92 in Form von zwei Zapfen 94, 94' vor, die dann in die entsprechenden Sacklöcher 76 und 77 der Interferenzschraube 70 eintreten können, wenn das Werkzeug 80 von proximal her auf die Interferenzschraube 70 aufgeschoben wird.

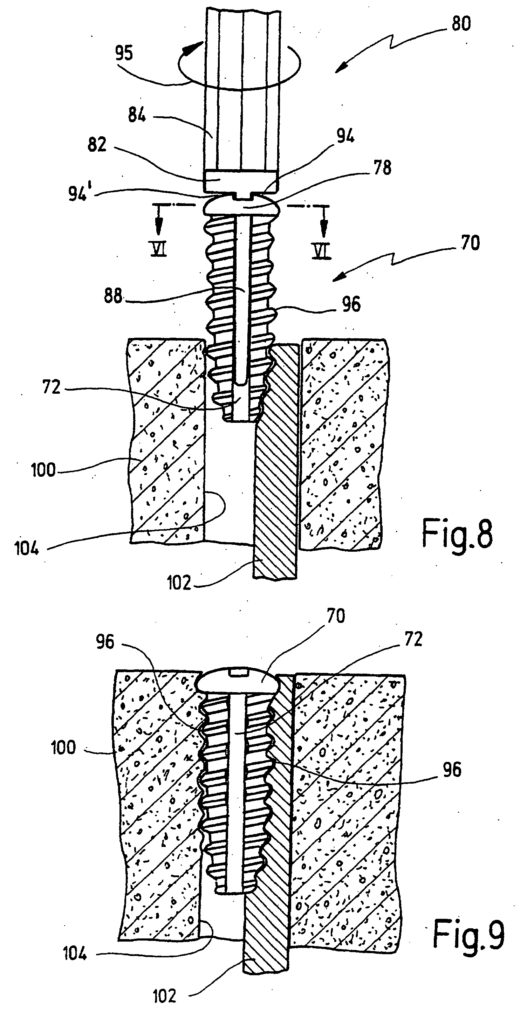

Eine solche Situation ist in Fig. 8 dargestellt. Daraus ist ersichtlich, daß das Werkzeug 80 so auf die Interferenzschraube 70 aufgesetzt ist, daß das Arbeitselement 88 in die Längsnut 72 von oben her eingeschoben ist und dieses vor seitlichem Austreten durch den Steg 78 geschützt ist. Bei vollständig aufgeschobenem Werkzeug 80 kommt dessen Unterseite 86 auf der Oberseite des Kopfes der Interferenzschraube 70 zum Liegen.

Aus der Darstellung von Fig. 8 ist zu erkennen, daß die Längsnut 72 über die gesamte axiale Länge der Interferenzschraube 70 eingeschnitten ist, also auch dessen Außengewinde 96 schneidet.

Der Vorgang einer Schraubenverankerung ist in der Folge von Fig. 8 und Fig. 9 dargestellt.

In dem Knochen 100, in den ein Sehnentransplantat 102 verankert werden soll, wird eine Öffnung in Form eines Bohrkanales 104 bewerkstelligt. Der Durchmesser des Bohrkanals 104 wird so gewählt, daß in diesen das Sehnentransplantat 102 bzw. ein Ende davon eingeschoben werden kann.

Bei Ersatz eines Kreuzbandes werden entsprechende Bohrkanäle sowohl im Femur als auch in der Tibia bewerkstelligt und dort jeweils das Sehnentransplantat verankert, wie das bspw. in Abbildung 1 des eingangs genannten Artikels in OP-Journal gezeigt ist.

Nach Einziehen des Sehnentransplantates 102 wird die Interferenzschraube 70 mit dem daran angesetzten Werkzeug 80 so angesetzt, daß sich deren Eintreibende zwischen die Innenwand des Bohrkanales 104 und das äußere Ende des Sehnentransplantates 102 schiebt. Dieses Einsetzen und Einschieben wird durch die Verjüngung im Bereich des Eintreibendes begünstigt.

Durch Drehen des Werkzeuges 80, wie das in Fig. 8 durch einen Pfeil 95 angedeutet ist, wird die Interferenzschraube eingedreht.

Dabei frißt sich das Außengewinde 96 in das Material des Knochens 100 ein und dringt auch in das relativ flexible Material des Sehnentransplantates 102 ein, ohne dieses zu verletzen oder zu zerstören und klemmt oder quetscht dieses in den verbleibenden Zwischenraum zwischen Interferenzschraube 70 und Bohrkanal 104.

Dadurch, daß das Arbeitselement 88 des Werkzeuges 80 die Außenkontur der Interferenzschraube 70 nicht überragt, wird dieses Eindrehen durch das an der Außenseite anliegende Werkzeug 80 nicht gehindert. Nach vollständigem Eindrehen der Interferenzschraube 70 kann das Werkzeug 80 einfach axial abgezogen werden, ohne daß dadurch die Lage oder der Sitz der Interferenzschraube 70 irgendwie verändert wird.

Diese Situation ist in Fig. 9 dargestellt, d.h. die Interferenzschraube 70 ist vollständig eingedreht und das Werkzeug 80 ist abgezogen.

Auch bei extrem kleinen und dünnen Interferenzschrauben ist es nun möglich, die erheblichen Kräfte zum Drehen der jeweiligen Interferenzschraube durch das in den Längsnuten anliegende Werkzeug zu übertragen, so daß die Interferenzschraube ohne Beeinträchtigung deren Stabilität oder Struktur an Ort und Stelle gebracht werden kann.

## Patentansprüche

1. Schraube für den medizinischen Einsatz, mit einem Schraubenkörper (12), der einen Kopfabschnitt (14) und einen daran anschließenden Schaftabschnitt (16) aufweist, welcher ein Außengewinde (22, 96) aufweist, und mit einer Ausnehmung (26), an der ein Werkzeug (40, 80) zum Drehen der Schraube (10, 60, 70) ansetzbar ist, wobei die Ausnehmung (26) in Form zumindest einer Längsnut (28, 29, 30; 62; 72) ausgebildet ist, die von der Außenseite (20) her in den Schraubenkörper (12) eingeschnitten ist, **dadurch gekennzeichnet, daß** sich die zumindest eine Längsnut (28, 29, 30; 62; 72) über die gesamte Länge des Schaftabschnitts (16) erstreckt, und daß am stirnseitigen Ende des Kopfabschnittes (14) zumindest eine Aussparung (32, 64, 74) vorgesehen ist, in die ein entsprechender Vorsprung (52, 92) am Werkzeug (40, 80) eintreten kann.

2. Schraube nach Anspruch 1, **dadurch gekennzeichnet, daß** die Einschnittiefe der zumindest einen Längsnut (28, 29, 30; 62; 72) derart ist, daß ein Arbeitselement (48, 49, 50; 88) des Werkzeuges (40, 80) innerhalb der Längsnut (28, 29, 30; 62; 72) zum Liegen kommt und umfänglich die Außenkontur der Schraube (10, 60, 70) nicht oder nur unwesentlich überragt.

3. Schraube nach Anspruch 1, **dadurch gekennzeichnet, daß** die Aussparung (32) als durch den Schraubenkörper (12) durchgehender Kanal (33) ausgebildet ist.

4. Schraube nach einem der Ansprüche 1 bis 3, **dadurch gekennzeichnet, daß** mehrere, gleichmäßig um den Außenumfang des Schraubenkörpers (12) verteilt angeordnete Längsnuten (28, 29, 30) vorgesehen sind.

5. Schraube nach Anspruch 4, **dadurch gekennzeichnet, daß** eine (62, 72) bis fünf Längsnuten, insbesondere drei Längsnuten (28, 29, 30) eingeschnitten sind.

6. Schraube nach einem der Ansprüche 1 bis 5, **dadurch gekennzeichnet, daß** eine jeweilige Längsnut (28, 29, 30; 62; 72) axial am stirnseitigen Ende des Kopfabschnittes (40) offen ist.

7. Schraube nach einem der Ansprüche 1 bis 6, **dadurch gekennzeichnet, daß** die Längsnut (72), insbesondere im Kopfabschnitt des Schraubenkörpers umfänglich mit zumindest einem Steg (78) überbrückt ist.

8. Schraube nach Anspruch 7, **dadurch gekennzeichnet, daß** der Steg durch das Außengewinde gebildet wird.

9. Schraube nach einem der Ansprüche 1 bis 8, **dadurch gekennzeichnet, daß** diese aus biodegradierbarem Material besteht.

10. Schraube nach einem der Ansprüche 1 bis 9, **dadurch gekennzeichnet, daß** diese als Interferenzschraube (10, 60, 70) zum Verankern eines Sehnentransplantates (102) in einer Öffnung in einem Knochen (100) ausgebildet ist.

11. Werkzeug (40, 80) zum Drehen einer Schraube (10, 60, 70) nach einem der Ansprüche 1 bis 10, **dadurch gekennzeichnet, daß** es zumindest ein Arbeitselement (48, 49, 50; 88) aufweist, das in eine entsprechende Längsnut (28, 29, 30; 72) an der Schraube (10, 60, 70) einsetzbar ist, und daß vom Werkzeug (40, 80) distalseitig zumindest ein Vorsprung (54, 92) vorsteht, der in die Aussparung (32, 64, 74) am stirnseitigen Kopfabschnitt der Schraube (10, 60, 70) eintreten kann.

12. Werkzeug nach Anspruch 11, **dadurch gekennzeichnet, daß** die Arbeitselemente (48, 49, 50; 88) derart ausgestaltet sind, daß diese in Längsrichtung von der Kopfseite der Schraube (10, 60, 70) her in eine entsprechende Längsnut (28, 29, 30; 62; 72) einschiebbar sind.

13. Werkzeug nach Anspruch 11 oder 12, **dadurch gekennzeichnet, daß** beim Vorhandensein von mehreren Arbeitselementen (48, 49, 50) diese untereinander über umfängliche Stege (56) miteinander verbunden sind.

14. Werkzeug nach Anspruch 13, **dadurch gekennzeichnet, daß** Sollbruchstellen zwischen den Arbeitselementen und den Stegen vorhanden sind.

## Claims

1. A screw for medical purposes comprising a screw body (12) having a head portion (14) and a following shaft portion (16) with an outer threading (22, 96) and a recess (26) on which a tool (40, 80) can be placed for rotating the screw (10, 60, 70), said recess (26) is configured of at least one axial groove (28, 29, 30; 62; 72) cut into the screw body (12) from the outside (20), **characterized in that** said at least one axial groove (28, 29, 30; 62; 72) extends over the entire length of the shaft portion (16) and **in that** at least one recess (32, 64, 74) is provided at the end face of the head portion (14), into which a corresponding projection (52, 92) of the tool (40, 80) can be introduced.

2. The screw of claim 1, **characterized in that** the depth of said at least one axial groove (28, 29, 30; 62; 22) is such that a working element (48, 49, 50; 88) of the tool (40, 80) lies within the axial groove (28, 29, 30; 62; 72) and does not extend or only negligibly extends beyond the periphery of the outer contour of the screw (10, 60, 70).

3. The screw of claim 1, **characterized in that** the recess (32) is configured as a channel (33) completely passing through the screw body (12).

4. The screw of anyone of claims 1 through 3, **characterized in that** several axial grooves (28, 29, 30) are provided to be distributed uniformly about the periphery of the screw body (12).

5. The screw of claim 4, **characterized in that** one (62, 72) to five axial grooves are cut therein, in particular three axial grooves (28, 29, 30).

6. The screw of anyone of claims 1 through 5, **characterized in that** each axial groove (28, 29, 30; 62; 72) is open axially at the end face of the head portion (40).

7. The screw of anyone of claims 1 through 6, **characterized in that** the axial groove (72) is cross-connected in circumferential direction with at least one bridge (78), particularly in the head portion of the screw body.

8. The screw of claim 7, **characterized in that** the bridge is formed by the outer threading.

9. The screw of anyone of claims 1 through 8, **characterized in that** it consists of biodegradable material.

10. The screw of anyone of claims 1 through 9, **characterized in that** it is configured as an interference screw (10, 60, 70) for anchoring a tendon transplant (102) in an opening in a bone (100).

11. A tool (40, 80) for rotating the screw (10, 60, 70) of anyone of claims 1 through 10, **characterized in that** it comprises at least one working element (48, 49, 50; 88) which can be placed in a corresponding axial groove (28, 29, 30; 72) on the screw (10, 60, 70) and **in that** at least one projection (54, 92) extends from the tool (40, 80) to the distal side, which can be introduced into the recess (32, 64, 74) on the end face of the head portion of the screw (10, 60, 70).

12. The tool of claim 11, **characterized in that** the working elements (48, 49, 50; 88) are configured such that they can be slid in longitudinal direction from the head of the screw (10, 60, 70) into a corresponding axial groove (28, 29, 30; 62; 72).

13. The tool of claims 11 or 12, **characterized in that** when several working elements (48, 49, 50) are present, these are cross-connected to one another by circumferential bridges (56).

14. The tool of claim 13, **characterized in that** breakage points are provided between the working elements and the bridges.

## Revendications

1. Vis pour utilisation médicale, avec un corps de vis (12) qui comporte une portion de tête (14) et une portion de tige (16) qui fait suite à celle-ci et qui présente un filetage extérieur (22, 96), et avec un évidement (26) contre lequel on peut appliquer un outil (40, 80) pour visser la vis (10, 60, 70), l'évidement (26) étant réalisé sous la forme d'au moins une rainure longitudinale (28, 29, 30; 62 ; 72) qui est entaillée à partir du côté extérieur (20) dans le corps de vis (12), **caractérisée en ce que** la ou les rainures longitudinales (28, 29, 30 ; 62 ; 72) s'étendent sur toute la longueur de la portion de tige (16), et **en ce qu'**à l'extrémité frontale de la portion de tête (14) est prévue au moins une découpe (32, 64, 74) dans laquelle peut pénétrer une saillie (52, 92) correspondante de l'outil (40, 80).

2. Vis selon la revendication 1, **caractérisée en ce que** la profondeur d'entaille de la ou des rainures longitudinales (28, 29, 30 ; 62 ; 72) est telle qu'un élément de travail (48, 49, 50 ; 88) de l'outil (40, 80) vient se trouver à l'intérieur de la rainure longitudinale (28, 29, 30 ; 62 ; 72) et ne dépasse pas périphériquement du contour extérieur de la vis (10, 60, 70) ou insensiblement seulement.

3. Vis selon la revendication 1, **caractérisé en ce que** la découpe (32) est réalisée en tant que canal (33) qui traverse de part en part le corps de vis (12).

4. Vis selon l'une des revendications 1 à 3, **caractérisée en ce que** sont prévues plusieurs rainures longitudinales (28, 29, 30) uniformément réparties autour du périmètre extérieur du corps de vis (12).

5. Vis selon la revendication 4, **caractérisée en ce qu'**une (62, 72) à cinq rainures longitudinales, en particulier trois rainures longitudinales (28, 29, 30) sont entaillées.

6. Vis selon l'une des revendications 1 à 5, **caractérisée en ce que** chaque rainure longitudinale (28, 29, 30 ; 62 ; 72) est ouverte axialement à l'extrémité frontale de la portion de tête (40).

7. Vis selon l'une des revendications 1 à 6, **caractérisée en ce que** la rainure longitudinale (82) est pontée périphériquement par au moins un pontet (78), en particulier dans la portion de tête du corps de vis.

8. Vis selon la revendication 7, **caractérisée en ce que** le pontet est formé par le filetage extérieur.

9. Vis selon l'une des revendications 1 à 8, **caractérisée en ce qu'**elle est constituée d'une matière biodégradable.

10. Vis selon l'une des revendications 1 à 9, **caractérisée en ce qu'**elle est réalisée en tant que vis d'interférence (10, 60, 70) pour l'ancrage d'un transplant de tendon (102) dans une ouverture d'un os (100).

11. Outil (40, 80) pour visser une vis (10, 60, 70) selon l'une des revendications 1 à 10, **caractérisé en ce qu'**il comporte au moins un élément de travail (48, 49, 50 ; 88) qui peut être inséré dans une rainure longitudinale (28, 29, 30 ; 72) correspondante de la vis (10, 60, 70), et **en ce que** de l'outil (40, 80) du côté distal dépasse au moins une saillie (54, 92) qui peut pénétrer dans la découpe (32, 64, 74) sur la portion de tête frontale de la vis (10, 60, 70).

12. Outil selon la revendication 11, **caractérisé en ce que** les éléments de travail (48, 49, 50 ; 88) sont conçus de manière que ceux-ci puissent être introduits dans la direction longitudinale, depuis le côté de tête de la vis (10, 60, 70), dans une rainure longitudinale (28, 29, 30 ; 62 ; 72) correspondante.

13. Outil selon la revendication 11 ou 12, **caractérisé en ce qu'**en présence de plusieurs éléments de travail (48, 49, 50), ceux-ci sont reliés entre eux par des nervures périphériques (56).

14. Outil selon la revendication 13, **caractérisé en ce que** des points destinés à la rupture sont prévus entre les éléments de travail et les nervures.
